# EUROPÄISCHE PATENTSCHRIFT

(11) **EP 2 239 040 B1**
(45) Veröffentlichungstag und Bekanntmachung des Hinweises auf die Patenterteilung: **22.01.2014**
(21) Anmeldenummer: 10168464.5
(22) Anmeldetag: 11.02.2004
(51) Int. Cl.: B01D 53/04, F17C 11/00

(54) **Gasspeicher und seine Verwendung zur Speicherung einer vorgegebenen Gasmenge und Freigabe des Gases für eine medizinische Applikation**
Gas storage device and use for storing an amount of gas and release of the gas for a medical application
Appareil pour le stockage d'un gaz et sa utilisation pour la décharge d'un quantité du gaz pour une application médicale

(30) Priorität: 15.02.2003 DE 10306344
(43) Veröffentlichungstag der Anmeldung: 13.10.2010
(62) Teilanmeldung aus: 04710020.1
(73) Patentinhaber: PHARMPUR GmbH, 86343 Königsbrunn (DE)
(72) Erfinder: Dr. Menz, Dirk Henning, 86420 Diedorf-Lettenbach (DE)
(74) Vertreter: Charrier, Rapp & Liebau

(56) Entgegenhaltungen:
- EP-A- 1 273 530
- WO-A-00/12196
- US-A- 4 023 701
- US-A- 6 073 759

## Beschreibung

Die Erfindung betrifft einen Gasspeicher zur Speicherung einer vorgegebenen Gasmenge eines perfluorierten Gases, sowie dessen Verwendung zur Sterilisierung des Gases und Freigabe des sterilisierten Gases für eine medizinische Applikation. Der Gasspeicher kann insbesondere für eine ophthalmologische Applikation des gespeicherten Gases eingesetzt werden, bspw. als intraokulare Gastamponade. Ein bekannter Gasspeicher ist in WO-A-0 012 196 beschrieben.

Gase finden in der Medizin vielfältige Anwendungen, wobei Beatmungs- oder Narkosegase besonders verbreitet sind. Darüber hinaus werden besonders perfluorierte Gase als Tamponademedien in der Ophtalmologie eingesetzt, wie beispielsweise in der DE 42 20 182 A1 beschrieben.

Sofern für derartige Anwendungen in der Medizin die Gase in größeren Mengen bereitzustellen sind, werden bekannte Mittel und Verfahren angewandt, beispielsweise zentrale Gasversorgungssysteme oder Druckgasflaschen. Werden Gase nur in geringen Mengen benötigt, so können diese in beutelartigen Behältern oder in Ballons aufbewahrt und bereitgestellt werden.

Bei medizinischen Anwendungen ist Sterilität der Gase erforderlich. Diese wird üblicherweise durch Filtration (Porengröße 0,2µ) erreicht.

Ein Nachteil von komprimierten Gasen ist, dass die bei der Kompression entstehenden Kondensate bzw. Ölverunreinigungen sicher beseitigt werden müssen. Dies stellt hohe Anforderungen an die verwendeten Kompressoren und Abscheidevorrichtungen.

Im Bereich der Ophtalmologie ist es bekannt, perfluorierte Gase wie SF₆, C₂F₆, C₃F₈, CF₄, entweder in reiner Form oder in Verdünnung mit Luft als vitrio-retinale Endotamponaden einzusetzen. Für diese Anwendungen genügen relativ kleine Gasmengen in der Größenordnung von einigen ml. Hierfür werden portionierte Gasmengen in Form von sogenannten Kit-Gas-Systemen bereitgestellt, bei denen die Gase in kunststoff-kaschierten Aluminiumbeuteln verpackt sind. Die Gasentnahme erfolgt über in den Beuteln integrierte Ventile oder LUER-Anschlüsse. Die erforderliche Sterilität der Gase wird unmittelbar vor ihrem Einsatz im Glaskörperraum durch eine 0,2µ Filtration sichergestellt.

Sowohl bei der Lagerung als auch insbesondere bei der Gasentnahme besteht die Gefahr, daß das zur Anwendung kommende Gas unkontrolliert mit Luft verdünnt wird. Verursacht wird dies einerseits durch Undichtigkeiten in den flexiblen Beuteln, beispielsweise durch Mikrorisse in der Beutelwand. Derartige Undichtigkeiten können herstellungsbedingt im Beutelmaterial enthalten sein oder durch Alterung oder unsachgemäße Lagerung entstehen. Andererseits kann ein unkontrolliertes Verdünnen des Tamponademediums mit Luft , bedingt durch unsachgemäße Bedienung bei der Gasentnahme, eintreten. Bei der Verwendung als vitrio-retinales Tamponadegas wird das Gas üblicherweise mit einer Spritze in den Glaskörperraum appliziert. Insbesondere beim Aufziehen der Spritze mit dem Tamponadegas kann das Eindringen von Fremdluft aufgrund des in der Spritze entstehenden Unterdrucks nicht sicher ausgeschlossen werden. Eine undefinierte Zusammensetzung des Tamponadegases, welche durch ein unkontrolliertes Verdünnen mit Luft hervorgerufen wird, oder eine Kontamination mit Mikroorganismen, die über die gleichen Wege wie Fremdluft in das Tamponadegas eindringen können, kann jedoch zu einer Gefährdung der Patienten führen.

Um die Tamponadegase in den bekannten Kit-Gas-Systemen im Operationssaal verwenden zu können, muss eine Außenhautsterilisation durchgeführt werden. In der Regel wird eine Außenhautsterilisation mit Wasserdampf bei 121° C durchgeführt. Bei diesem Sterilisationsvorgang erhöht sich aber der Druck in dem flexiblen Verpackungsbeutel um etwa einen Faktor 5. Dies führt zu extremen Belastungen des Verpackungsmaterials, weshalb es zu Undichtigkeiten kommen kann. Eine Strahlensterilisation scheidet als Möglichkeit ebenso aus, da während der Bestrahlung von Fluorcarbonen Radikale entstehen. Deshalb wird bei den Kit-Gas-Systemen üblicherweise mit EO sterilisiert.

Der vorliegenden Erfindung liegt daher die Aufgabe zugrunde, die Verwendung eines Gasspeichers zur Speicherung einer Gasmenge und Freigabe des Gases für eine medizinische Applikation aufzuzeigen, bei der sowohl bei der Lagerung als auch bei der Gasentnahme ein unkontrolliertes Vermischen des Gases mit Umgebungsluft oder Fremdgasen vermieden wird. Der Gasspeicher soll ferner eine einfache und zuverlässige Sterilisierung des Gases ermöglichen.

Gelöst werden diese Aufgaben mit einem Gasspeicher mit den Merkmalen des Anspruchs 1 sowie dessen Verwendung nach Anspruch 7 Vorteilhafte Ausführungsbeispiele des Gasspeichers und dessen Verwendung sind den abhängigen Ansprüchen zu entnehmen.

Gemäß der Erfindung umfaßt der Gasspeicher einen gasdichten Behälter, in dem ein Adsorbens enthalten ist, an dem bei Raumtemperatur eine vorgegebene Gasmenge eines perfluorierten Gases sowie Wasser adsorbiert ist, wobei es sich bei dem Adsorbens um ein Zeolith handelt. Durch Erhitzen des Gasspeichers auf bspw. Temperaturen von 121°C oder mehr ist das am Adsorberns adsorbierte Gas sterilisierbar und kann nach dem Sterilisieren durch Desorption für die vorgesehene medizinische Applikation, wie bspw. eine ophthalmologische Anwendung z.B. als intraokulare Gastamponade, frei gegeben werden.

Durch die Adsorbtion des Gases an dem Adsorbens wird bei der Lagerung des Gasspeichers ein unkontrolliertes Vermischen mit Umgebungsluft verhindert. Darüber hinaus wird bei diesem Gasspeicher auch bei der Gasentnahme ein eindiffundieren von Umgebungsluft vermieden, da die Entnahme des Gases nicht wie bei den bekannten Kit Gas Systemen durch Erzeugung eines Unterdrucks erfolgt, sondern durch Überdruck, der entsteht, indem das adsorbierte Gas bei Erwärmung vom Adsorbens desorbiert.

In einem bevorzugten Ausführungsbeispiel erfolgt die Desorption des Gases durch Erwärmung des Adsorbens auf Temperaturen oberhalb der Gas-Desorptionstemperatur. In einem alternativen Ausführungsbeispiel erfolgt die Desorption des Gases aufgrund einer Verdrängungsreaktion, welche durch Adsorption eines Stoffes mit einer höheren Adsorptionsaffinität als das zur medizinischen Applikation vorgesehene Gas an das Adsorbens eingeleitet wird.

Bei dem erfindungsgemäßen Gasspeicher bzw. dessen erfindungsgemäßer Verwendung ist neben dem Gas für die medizinische Applikation noch Wasser bzw. Wasserdampf am Adsorbens adsorbiert. Dadurch kann das Gas durch Erhitzen des Gasspeichers auf Temperaturen von bspw. mindestens 121 °C sterilisiert werden. Durch die dadurch erfolgende Erhitzung des adsorbierten Wassers bzw. Wasserdampfs werden sämtliche Keime im Gasvolumen abgetötet, wodurch sowohl das adsorbierte Gas als auch das Adsorbens sterilisiert wird.

Bei dem erfindungsgemäßen Gasspeicher ist das Adsorbens in einem verschlossenen Behälter unter Atmosphärendruck untergebracht, wobei. es sich bei dem Adsorbens um ein Zeolith handelt. Diese Zeolithe müssen bevorzugt so ausgewählt sein, dass sie eine abgestimmte Adsorptions- und Desorptinsgeschwindigkeit für Gase besitzen. Einerseits darf die Desorption weder bei Raumtemperatur beginnen noch darf aus Sicherheitsgründen durch Desorption bei 121°C in geschlossenen Gefäßen ein Druckanstieg über 5 bar erfolgen. Andererseits muß die Desorption zügig bei Temperaturen erfolgen, bei denen keine Zerstörung von Kunststoffmaterialien auftritt. Für unpolare Gase sind Zeolithe aus der Gruppe der dealuminierten Zeolithe, vorzugsweise Y Zeolithe mit einem SiO2/Al2O3-Verhältnis > 200, insbesondere mit einem SiO2/Al2O3-Verhältnis > 1000, dafür geeignet. Ebenso sind Pentasile, das sind Zeolithe aus der Gruppe der dealuminierten Zeolithe mit einem SiO2/Al2O3-Verhätnis > 250, insbesondere mit einem SiO2/Al2O3-Verhältnis > 1000, dafür geeignet. Geeignet sind auch Zeolithe der MORDENITE-Gruppe mit einem SiO2/Al2O3-Verhältnis von 15-20. Für den bestimmungsgemäßen Gebrauch sind Porengrößen von vorzugsweise 0,2-1 nm und Langmuiroberflächen > 400 m²/g zu nutzen.

Bei einer bevorzugten Anwendung des Gasspeichers zur Freigabe des Gases für eine ophtalmologische Applikation handelt es sich bei dem gespeicherten Gas um SF₆, CF₄, C₂F₆ oder C₃F₈-Gas oder um ein Gemisch dieser Gase. Diese Gase können als intraokulare Gastamponade in den Glaskörperraum appliziert werden.

Um das Gas für die vorgesehene medizinische Applikation freizusetzen wird das Gas vom Adsorbens desorbiert und in ein Applikationsmittel, welches bevorzugt eine Einwegspritze ist, eingebracht. In einem besonders bevorzugten Ausführungsbeispiel erfolgt das Einbringen des Gases in die Einwegspritze selbsttätig, d. h. ohne daß die Spritze manuell aufgezogen werden müßte.

Eine weitere Ausführungsform der Erfindung gewährleistet eine möglichst einfache Handhabung des Gasspeichers, indem das Adsorbens als Formkörper vorliegt.

Die erfindungsgemäße Verwendung des Gasspeichers erlaubt auch die Bereitstellung eines hochgereinigten Gases für eine medizinische Anwendung. Dazu wird ein Gasspeicher bereitgestellt, der ein Adsorbens umfasst, an dem bei Raumtemperatur die vorgegebene Gasmenge und etwaige Verunreinigungen adsorbiert sind. Das Adsorbens ist hierbei mit einer Flüssigkeit getränkt, in der das für die Anwendung vorgesehene Gas und die vorhandenen Verunreinigungen eine unterschiedliche Löslichkeit aufweisen. Zur Freigabe des Gases für eine medizinische Anwendung wird ein Desorptionsvorgang durchgeführt, bei dem aufgrund der unterschiedlichen Löslichkeiten des Gases bzw. der Verunreinigungen zunächst die in der Flüssigkeit nicht bzw. schlechter löslichen Komponenten und danach zeitlich versetzt die besser löslichen Komponenten desorbieren. Hierdurch kann erreicht werden, dass bei der Desorption zunächst die in der Flüssigkeit nicht bzw. schlechter löslichen Verunreinigungen desorbieren, welche dann abgetrennt werden, und erst danach eine Desorption des sich in der Flüssigkeit besser löslichen Gases erfolgt. Im umgekehrten Fall, wenn sich das Gas in der Flüssigkeit schlechter löst als die Verunreinigungen, kann zunächst die Desorption und Entnahme des zur Anwendung vorgesehenen Gases erfolgen, wobei die in der Flüssigkeit löslichen Verunreinigungen am Adsorbens adsorbiert bleiben.

Ein wesentlicher Vorteil des erfindungsgemäßen Gasspeichers besteht auch darin, dass die nicht mehr benötigte Gasmenge leicht recycled werden kann. Werden Gase portioniert bereitgestellt, so muß die Packungsgröße so bemessen sein, dass die vorgesehene Gasmenge sicher entnommen werden kann. Deshalb wird mindestens die doppelte Gasmenge, in der Regel das drei- bis vierfache dieser Menge, bereitgestellt. Der sich daraus ergebende Überschuß entweicht bei Verwendung handelsüblicher Kits unkontrolliert in die Atmosphäre. Aus Umweltschutzgründen sollte dies vermieden werden. Dies kann nun bei dem erfindungsgemäßen Gasspeicher bzw. dessen erfindungsgemäßer Verwendung leicht erreicht werden, wenn nach Entnahme der gewünschten Gasmenge das Vorratsgefäß abgekühlt wird. Dann erfolgt eine Re-Adsorption der Restgasmenge. Die Gasspeicher können dann leicht einem Recycling bzw. einer kontrollierten Entsorgung zugeführt werden.

Nachfolgend wird die Erfindung anhand von Ausführungsbeispielen näher erläutert.

Bei der Herstellung des Gasspeichers wird zunächst ein geeignetes Adsorber-Material, beispielsweise Zeolith, bei Temperaturen von ca.100- 120° C unter Vakuum ausgeheizt um etwaige Verunreinigungen vom Adsorber-Material zu entfernen. Anschließend erfolgt eine Adsorbtion eines für eine medizinische Applikation vorgesehenen Gases an das Adsorbens. Bei dem für die medizinische Applikation vorgesehenen Gas kann es sich beispielsweise um für eine vitrio-retinale Gastomponade geeignete perfluorierte Gase wie SF₆, C₂F₆, CF₄ oder C₃F₈ handeln. Bei diesen Gasen haben sich insbesondere Zeolithe mit einem hohen Si/Al-Verhältnis als geeignete Adsorber herausgestellt, weil diese Zeolithe (DAZ- bzw. DAY-Zeolithe) geeignet sind große Mengen dieser Gase bei kleinem Adsorbens-Volumen zu speichern. Um eine möglichst einfache Handhabung des Gasspeichers zu gewährleisten, liegt das Adsorbens als Formkörper vor.

Die Adsorption des jeweiligen Gases erfolgt durch übliche Verfahren, beispielsweise das Überströmen des Adsorbens mit dem jeweiligen Gas bei Raumtemperatur. Die adsorbierte Menge des Gases entspricht der für die jeweils vorgesehene Anwendung benötigten Gasmenge. Das Gasadsorbat kann wie Schüttgut gehandhabt werden. Gleichzeitig oder zeitlich versetzt wird neben dem Gas Wasser oder Wasserdampf an das Adsorbens adsorbiert. Die Menge des adsorbierten Wassers bzw. Wasserdampfs wird hierbei so gewählt, dass sie ausreichend ist, das adsorbierte Gas durch Erhitzen des Gasspeichers auf Temperaturen von mindestens 121 ° C zu sterilisieren. Das mit dem Gas und Wasser bzw. Wasserdampf adsorbierte Adsorbens wird anschließend in einen gasdichten Behälter, beispielsweise eine Glasflasche mit einem Septum und Aluminium-Verschluß eingebracht. Anschließend erfolgt ein Sterilisieren des Gases durch Erhitzen des Behälters auf Temperaturen von mindestens 121 ° C. Die Erhitzung kann entweder trocken oder bevorzugt durch Dampfsterilisation im Autoklaven erfolgen, wobei bei der Dampfsterilisation im Autoklaven nicht nur das adsorbierte Gas und das Adsorbens sondern auch die Außenhaut des Behälters sterilisiert wird. Durch Erhitzen des Gasspeichers auf Temperaturen von mindestens 121° C werden zumindest innerhalb des gasdichten Behälters sämtliche Keime durch die sich im Behälter bildende Wasserdampfatmosphäre abgetötet.

Mit dem so hergestellten Gasspeicher wird eine vorbestimmte Menge des zur medizinischen Applikation vorgesehenen Gases bereitgestellt. Zur Freisetzung des Gases wird der Behälter zunächst auf Temperaturen oberhalb der Desorptionstemperatur des Gases erhitzt und anschließend dem Behälter das Gas entnommen. Zur medizinischen Applikation des Gases wird dieses einem Applikationsmittel, beispielsweise einer Einwegspritze, zugeführt. Eine solche gasdichte Spritze kann dabei auf dem üblichen Weg, d. h. nach dem Durchstechen eines geeigneten Septums, das den Behälter gasdicht verschließt, mit dem Gas befüllt werden. Alternativ kann der Behälter vor dem Desorptionsschritt mit einem Entnahmesystem verbunden werden, welches das Andocken einer Spritze oder eines anderen Applikationsmittels ermöglicht. Üblicherweise werden dazu Luer-Anschlüsse verwendet. Indem auf diese Weise eine direkte Verbindung des Applikationsmittels mit dem Innenraum des zu erhitzenden Behälters erfolgt, kann das desorbierte Gas aufgrund des vorhandenen Überdrucks in das Applikationsmittel (bspw. in die Spritze) eindringen. Es erfolgt also bspw. ein selbsttätiges Befüllen der Spritze, indem das desorbierte Gas den Kolben der Spritze bewegt, ohne daß die Spritze manuell aufgezogen werden müßte. Der Erfolg eines Füllllvorganges kann also direkt beobachtet und kontrolliert werden. Das so mit Gas befüllte Applikationsmittel kann dann anschließend der vorgesehenen Verwendung, beispielsweise als Gastamponade in den Glaskörperraum des Auges, zugeführt werden.

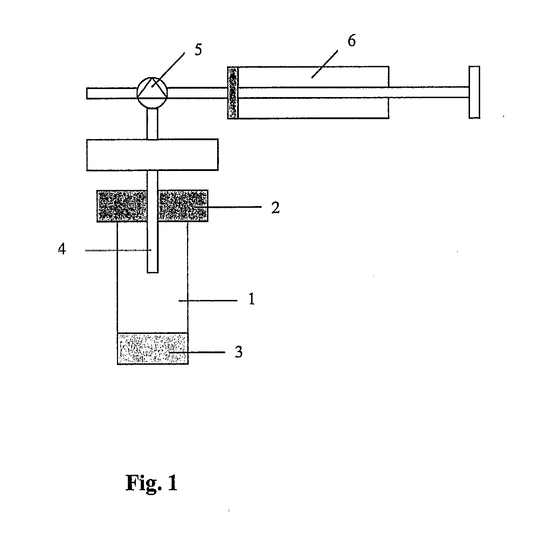

In der **Figur 1** ist ein erfindungsgemäßer Gasspeicher zusammen mit einer Gasentnahmevorrichtung schematisch dargestellt. Der Gasspeicher umfaßt eine Glasflasche 1, welche mit einem Septum und Aluminium-Verschluß 2 verschlossen ist. In der Glasflasche 1 ist das Adsorber-Material 3 eingebracht. Durch den Aluminium-Verschluß mit Septum 2 ist ein Entnahmeröhrchen 4 geführt. Dieses Entnahmeröhrchen ist über ein Ventil 5 bzw. über einen Dreiwegehahn mit einer Spritze 6 verbunden. Die Befüllung der Spritze 6 erfolgt selbsttätig in der oben beschriebenen Weise durch Erwärmung der Glasflasche 1 auf Temperaturen oberhalb der Desorptionstemperatur des Gases.

Ein unkontrolliertes Vermischen des jeweiligen Gases wird sowohl bei der Lagerung als auch bei der Gasentnahme zuverlässig vermieden. Bei der Gasentnahme ist ein unkontrolliertes Eindringen von Umgebungsluft aufgrund des Überdrucks des desorbierten Gases ausgeschlossen. Gegebenenfalls vorhandene Lecks im Applikationsmittel führen ebenfalls nicht zu einem unerwünschten Eindringen von Umgebungsluft, weil das desorbierte Gas auch in Lecköffnungen eindringt und dort ein Gaspolster bildet, welches das Eindringen von Luft verhindert. Bei Verwendung eines Entnahmesystems kann darüber hinaus noch ein Spülvorgang zwischengeschaltet werden, der einerseits zur Dichtigkeitsprüfung genutzt werden kann. Andererseits wird dadurch sicher das gesamte Restgas aus dem System entfernt.

Da sowohl die Herstellung des Gasspeichers als auch die Montage der Gasentnahme jeweils bei Raumtemperatur erfolgt, bleiben die am Adsorbens adsorbierten Gase fest mit diesem verbunden. Erst durch die Desorption des Gases, z.B. durch Erhitzen des Adsorbens auf Temperaturen oberhalb der Desorptionstemperatur, erfolgt eine Freisetzung des Gases. Somit ist es möglich, vor der Gasentnahme in drucklosem Zustand alle Anschlüsse zu montieren und auf Dichtigkeit zu prüfen, ohne dass dafür eine bestimmte Reihenfolge der Arbeiten zwingend eingehalten werden muss.

In einem besonders bevorzugten Ausführungsbeispiel wird zunächst ca. 1 g dealuminiertes Zeolith mit einem SiO2/Al2O3 Verhältnis >1000 bei 120 °C unter Vakuum aktiviert und anschließend in ein 20 ml Headspacegläschen mit Sicherheitsverschluß überführt. Der Zugang zum Inneren des Gläschens wird durch zwei Kanülen ermöglicht. Durch diese wird dann C3F8 bis zur Gewichtskonstanz über das Adsorbens geleitet. Danach wird das Gläschen geöffnet, 0,2 ml Wasser (WFI Qualität) und ein Testkeimstreifen hinzugefügt und das Gläschen anschließend mit einem neuen Septum verschlossen. Die danach durchgeführte Behandlung im Dampfsterilisator bei 121°C führt zur vollständigen Abtötung der Keime.

| Verwendete Keimstreifen/Testkeim | Bac. stearothermophilus, ATCC 7953 (Simicon) |
|---|---|
| Population vor der Behandlung | 10⁶ |
| Keimzahl nach der Behandlung | <1 |

Bei der Gasentnahme wird der Gasspeicher auf eine Temperatur erhitzt, welche oberhalb der Desorptionstemperatur des Gases, aber unterhalb der Desorptionstemperatur von Wasser liegt. Auf diese Weise ist bei der Gasentnahme gewährleistet, daß der Wasserdampfpartialdruck die Entnahme des Gases nicht beeinflußt. Um den gleichen Effekt zu erzielen, können auch zwei unterschiedliche Adsorbertypen zum Einsatz kommen, wobei der eine Adsorbertyp das für die medizinische Applikation vorgesehene Gas nicht oder nur schlecht adsorbiert, dagegen gute Adsorption von Wasser gewährleistet und der andere Adsorbertyp das Gas gut adsorbiert, nicht hingegen Wasser.

Dieses Prinzip der stufenweisen Freisetzung von Gasen kann auch zu einer zeitgleich mit der Desorption erfolgenden Reinigung des Gases benutzt werden. Hierzu müssen die jeweils unterschiedlichen Adsorbtions- bzw. Desorptionstemperaturen so genutzt werden, daß die Gase möglichst vollständig voneinander getrennt freigesetzt werden.

In einer weiteren Ausführungsform der Erfindung wird ein Gasspeicher zur Speicherung einer vorgegebenen Gasmenge und Freigabe des Gases in möglichst reiner Form, also ohne Verunreinigungen, bereitgestellt. Der Gasspeicher umfasst ein Adsorbens, an dem bei Raumtemperatur Adsorbatkomponenten, nämlich die vorgegebene Gasmenge und etwaige Verunreinigungen adsorbiert sind. Das Adsorbens ist hierbei mit einer Flüssigkeit getränkt, in der das für die Anwendung vorgesehene Gas und die vorhandenen Verunreinigungen eine unterschiedliche Löslichkeit aufweisen. Hierdurch wird erreicht, dass bei einem Desorptionsvorgang zur Freigabe des Gases zunächst die in der Flüssigkeit nicht bzw. schlechter löslichen Adsorbatkomponenten, also die vorgegebene Gasmenge oder die Verunreinigungen, und erst danach zeitlich versetzt die besser löslichen Adsorbatkomponenten, also die Verunreinigungen oder die vorgegebene Gasmenge, desorbiert. Als geeignete Flüssigkeiten zur Tränkung des Adsorbens haben sich bei Raumtemperatur flüssige Fluorcarbone erwiesen. Aus mit flüssigen Fluorcarbonen getränkten Zeolithen werden nämlich bei einem Desorptionsvorgang zuerst die nicht in den Fluorcarbonen löslichen Gasverunreinigungen, wie z.B. Äthylenoxid oder nicht-fluorierte Kohlenwasserstoffe, abgegeben. Diese können somit zunächst durch Spülen aus dem System entfernt werden, bevor die Freigabe der zur Anwendung vorgesehenen Gasmenge erfolgt. Die zur Anwendung vorgesehenen Gase lösen sich dagegen im flüssigen Fluorcarbon auf und werden deshalb zunächst zurückgehalten und erst im späteren Verlauf des Desorptionsvorgangs mit zeitlicher Verzögerung freigesetzt. Die Freisetzung des für die Anwendung vorgesehenen Gases erfolgt erst dann, wenn die flüssigen Fluorcarbone mit dem jeweiligen Gas gesättigt sind. Auf diese Weise kann bei der Desorption eine Reinigung des zur Anwendung vorgesehenen Gases durch Ausnutzen der Desorptions- und Löslichkeitsunterschiede erreicht werden. Als geeignete Flüssigkeiten zur Tränkung des Adsorbens haben sich insbesondere Fluorcarbone, wie z.B. Perfluorperhydrophenantren, oder auch Kohlenwasserstoffe mit hohem Siedepunkt und geringem Dampfdruck erwiesen.

Bei der Verwendung eines mit einem Fluorcarbon getränkten Adsorbens lösen sich fluorierte Kohlenwasserstoffe in der Flourcarbon-Flüssigkeit und werden bei der Desorption zunächst zurückgehalten, bis die schlechter in der Fluorcarbon-Flüssigkeit löslichen Verunreinigungen desorbiert sind. Die Entnahme des zur Applikation vorgesehenen Gases aus dem Behälter erfolgt erst, nachdem sämtliche Verunreinigungen desorbiert sind.

Bei einem mit einem hochsiedenden Kohlenwasserstoff mit niedrigem Dampfdruck getränkten Adsorbens lösen sich Kohlenwasserstoffgase gut in dieser Tränkflüssigkeit. Etwaige Kohlenwasserstoff-Verunreinigungen werden dementsprechend bei der Desorption zunächst zurückgehalten. Um für die vorgesehene Applikation ein hochgereinigtes Gas aus dem Behälter entnehmen zu können, ist deshalb erforderlich, dass die Entnahme des zur Applikation vorgesehenen Gases gestoppt wird, bevor auch die Kohlenwasserstoff Verunreinigungen vom Adsorbens desorbeiren.

In einer weiteren Ausführungsform der Erfindung wird die Adsorptionswärme, die beim Adsorbieren von Stoffen am Adsorber entsteht, als Wärmequelle zur thermischen Desorption des zur medizinischen Applikation vorgesehenen Gases genutzt. Hierfür wird beispielsweise Wasser mit dem mit Gas adsorbierten Zeolith in Kontakt gebracht, wodurch aufgrund der Wasseradsorption am Zeolith Adsorptionswärme frei wird. Wenn die frei gesetzte Adsorptionswärme das Adsorbens bis über die Desorptionstemperatur des Gases erwärmt wird, erfolgt eine Desorption des Gases.

In einem alternativen Ausführungsbeispiel kann die Desorption des Gases auch durch einen Verdrängungsmechanismus bewirkt werden. Hierfür werden Stoffe am Adsorber angelagert, welche eine größere Adsorptionsneigung als das zur medizinischen Applikation vorgesehene Gas aufweisen. Als hierfür geeignete Stoffe haben sich perfluorierte Verbindungen erwiesen. Werden diese Verbindungen in den Behälter mit dem Gasadsorbat eingeleitet, findet eine Adsorption dieser Verbindungen am Adsorber und gleichzeitig aufgrund einer Verdrängungsreaktion eine Desorption des zur medizinischen Applikation vorgesehenen Gases statt.

Die nachfolgend dargestellte Zusammenfassung von Versuchsergebnissen sollen die Ausführungsbeispiele weiter verdeutlichen.

### Allgemeine Versuchsführung:

1. Nach Aktivierung der in der folgenden Tabelle aufgeführten Zeolithe über eine Stunde bei 120° C und 65 mbar wurden die aufgeführten Gase bei Raumtemperatur über die Adsorbentien bis zur Gewichtskonstanz bei Raumtemperatur übergeleitet. Dafür wurden die Adsorbentien (Zeolithe) in Sicherheitsgläschen, die mit einem Sicherheitsverschluß verschlossen waren, vorgelegt. Nach dem Ankoppeln einer pneumatischen Wanne wurden die Glasfläschen stufenweise erhitzt und die desorbierten Gase aufgefangen. Die desorbierte Gasmenge wurde volumetrisch bestimmt.
Ergebnisse:

| Absorbens | Gas | Aufnahme Gas in m % | Temperaturbereich der Desorption in °C | Rückgewinnung bei 100°C in % |
|---|---|---|---|---|
| Silikalit | C3F8 | 21 | 60-100 | 33 |
| MSM20 | C3F8 | 19 | 40-100 | 45 |
| SN300 | | 10 | 40-100 | 36 |
| DAZ | | 13 | 60-80 | 34 |
| DAZ + 5ml Perfluoroctan | | | | 59 |
| DAZ | SF6 | 22 | 50-75 | 63 |
| DAZ + 5ml Perfluoroctan | | | | 100 |

| Absorbens | Gas | Druck in kPa innerhalb Sicherheitsgläschen bei 100°C |
|---|---|---|
| DAZ | C3F8 | 92 |
| DAZ | SF6 | 230 |

Wird die Desorptionstemperatur erhöht, können die adsorbierten Gase vollständig zurückgewonnen werden.
2. Wie unter 1. beschrieben, wurden die Adsorbentien beladen. Anschließend wurden die Gläschen geöffnet und unter Raumbedingungen gelagert. Dabei wurde die Veränderung der Masse bestimmt.

| Absorbens | Gas | Masseabnahme bei Raumbedingungen nach 2Tagen im geöffneten Gläschen in % |
|---|---|---|
| DAZ | C3F8 | 2 |
| DAZ | SF6 | 5 |

## Patentansprüche

1. Gasspeicher zur Speicherung einer vorgegebenen Gasmenge eines perfluorierten Gases, umfassend einen gasdichten Behälter, in dem ein Adsorbens eingebracht ist, wobei das Adsorbens ein Zeolith ist, an dem bei Raumtemperatur die vorgegebene Gasmenge des perfluorierten Gases sowie Wasser adsorbiert ist.

2. Gasspeicher nach Anspruch 1, **dadurch gekennzeichnet, dass** das Adsorbens ein Zeolith aus der Gruppe der dealuminierten Zeolithe ist, vorzugsweise ein Zeolith Y mit einem SiO2/Al2O3-Verhältnis > 200 insbesondere mit einem SiO2/Al2O3 Verhältnis > 1000.

3. Gasspeicher nach einem der Ansprüche 1 oder 2, **dadurch gekennzeichnet, dass** das Adsorbens ein Zeolith aus der Gruppe der MORDENITE mit einem SiO2/Al2O3-Verhältnis von 15-20 ist.

4. Gasspeicher nach einem der Ansprüche 1 bis 3, **dadurch gekennzeichnet, dass** die Adsorbentien Porengrößen von 0,2-1 nm und Langmuiroberflächen > 400 m²/g aufweisen.

5. Gasspeicher nach einem der Ansprüche 1 bis 4, **dadurch gekennzeichnet, dass** das gespeicherte Gas ein perfluoriertes Gas aus der Gruppe SF₆, CF₄, C₂F₆ oder C₃F₈ oder ein Gemisch dieser Gase ist.

6. Gasspeichers nach einem der Ansprüche 1 bis 5, **dadurch gekennzeichnet, dass** die Desorptionstemperatur des Gases niedriger ist als die Desorptionstemperatur von H₂O.

7. Verwendung eines Gasspeichers nach einem der voranstehenden Ansprüche zur Sterilisierung eines gespeicherten perfluorierten Gases und Freigabe des sterilisierten Gases für eine medizinische Applikation, wobei das adsorbierte Gas durch Erhitzen des Gasspeichers sterilisiert und anschließend durch Desorption frei gegeben wird.

8. Verwendung eines Gasspeichers nach Anspruch 7, **dadurch gekennzeichnet, dass** der Desorptionsdruck des mit Gas beladenen Adsorbens bei 121°C 3,5 bar in einem verschlossenen Behälter nicht übersteigt, wenn der Behälter zur Hälfte seines Volumens mit dem Adsorbens gefüllt ist.

9. Verwendung nach einem der Ansprüche 7 oder 8, **dadurch gekennzeichnet, dass** die Desorption des Gases durch Erwärmung des Adsorbens über die Desorptionstemperatur des Gases erfolgt.

10. Verwendung nach einem der Ansprüche 7 bis 9, **dadurch gekennzeichnet, dass** das Gas für die vorgesehene medizinische Applikation durch Erwärmung freigesetzt und in ein Applikationsmittel eingebracht wird.

11. Verwendung nach einem der Ansprüche 7 bis 10 , **dadurch gekennzeichnet, dass** die Freigabe des adsorbierten Gases durch Adsorption eines Stoffes mit einer höheren Adsorptionsaffinität als das adsorbierte Gas an das Adsorbens erfolgt, wodurch eine Verdrängungsdesorption des Gases eingeleitet wird.

12. Verwendung nach einem der Ansprüche 7 bis 11, **dadurch gekennzeichnet, dass** das Gas durch thermische Desorption vom Adsorber desorbiert wird, indem ein Zusatzstoff in den Gasspeicher eingeleitet wird, der am Adsorber adsorbiert, wodurch eine solche Menge an Adsorptionswärme frei gesetzt wird, welche zur Desorption des Gases führt.

13. Verwendung nach einem der Ansprüche 7 bis 12, **dadurch gekennzeichnet, dass** zur Gasentnahme das Gas durch Verdrängungsdesorption vom Adsorber desorbiert wird, indem ein Zusatzstoff in den Gasspeicher eingeleitet wird, der zum verwendeten Adsorbens eine höhere Adsorptionsaffinität als das zur medizinischen Applikation vorgesehene Gas aufweist.

14. Verwendung nach einem der Ansprüche 7 bis 13, **gekennzeichnet durch** folgende Schritte:
- Ausheizen des Adsorbens um Verunreinigungen zu entfernen und um dieses zu aktivieren,
- Adsorption des Gases an das Adsorbens und Zugabe von Wasser
- Verschliessen des mit dem Gas adsorbierten Adsorbens in einem gasdichten Behälter,
- Sterilisieren des Gases und des Adsorbers in dem Behälter **durch** Erhitzen des Behälters auf Temperaturen von mindestens 121°C.

15. Verfahren zur Bereitstellung einer vorgegebenen Menge eines perfluorierten Gases für eine medizinische Applikation aus einem Gasspeicher nach einem der Ansprüche 1 bis 6, wobei die am Adsorbens adsorbierte Gasmenge durch thermische Desorption oder Verdrängungsdesorption von dem im Gasspeicher enthaltenen Adsorbens freigesetzt wird.

## Claims

1. Gas storage device for storage of a predetermined gas amount of a perfluorinated gas, comprising a gas-tight container in which an adsorbent is introduced, wherein the adsorbent is a zeolite on which the predetermined gas amount of the perfluorinated gas and water are adsorbed.

2. Gas storage device according to claim 1, **characterized in that** the adsorbent is a zeolite from the group of dealuminized zeolites, preferably a zeolite Y having an SiO₂/Al₂O₃ ratio of > 200, in particular having an SiO₂/Al₂O₃ ratio of > 1,000.

3. Gas storage device according to one of claims 1 or 2, **characterized in that** the adsorbent is a zeolite from the group of MORDENITES having an SiO₂/Al₂O₃ ratio of 15 - 20.

4. Gas storage device according to one of claims 1 to 3, **characterized in that** the adsorbents have pore sizes of 0.2 - 1 nm and Langmuir surface areas of > 400 m²/g.

5. Gas storage device according to one of claims 1 to 4, **characterized in that** the stored gas is a perfluorinated gas from the group of SF₆, CF₄, C₂F₆ or C₃F₈ or a mixture of these gases.

6. Gas storage device according to one of claims 1 to 5, **characterized in that** the desorption temperature of the gas is lower than the desorption temperature of H₂O.

7. Use of a gas storage device according to one of the preceding claims for sterilizing a stored perfluorinated gas and releasing the sterilized gas for a medical application, wherein the adsorbed gas is sterilized by heating the gas storage device and is then released by desorption.

8. Use of a gas storage device according to claim 7, **characterized in that** the desorption pressure at 121 °C of the adsorbent loaded with gas does not exceed 3.5 bar in a closed container when the container is filled with the adsorbent to half its volume.

9. Use according to one of claims 7 or 8, **characterized in that** the desorption of the gas is carried out by heating the adsorbent above the desorption temperature of the gas.

10. Use according to one of claims 7 to 9, **characterized in that** the gas is released for the envisaged medical application by heating and is introduced into an application means.

11. Use according to one of claims 7 to 10, **characterized in that** the release of the adsorbed gas is carried out by adsorption of a substance having a higher adsorption affinity for the adsorbent than the adsorbed gas, as a result of which a displacement desorption of the gas is initiated.

12. Use according to one of claims 7 to 11, **characterized in that** the gas is desorbed from the adsorber by thermal desorption, **in that** an additive which is adsorbed on the adsorber is introduced into the gas storage device, as a result of which such an amount of heat of adsorption which leads to desorption of the gas is released.

13. Use according to one of claims 7 to 12, **characterized in that** for withdrawal of the gas, the gas is desorbed from the adsorber by displacement desorption in that an additive which has a higher adsorption affinity for the adsorbent used than the gas envisaged for the medical application is passed into the gas storage device.

14. Use according to one of claims 7 to 13, **characterized by** the following steps:
- thorough heating of the adsorbent in order to remove impurities and in order to activate this
- adsorption of the gas on to the adsorbent and addition of water
- closing of the adsorbent on which the gas is adsorbed in a gas-tight container
- sterilizing of the gas and the adsorber in the container by heating the container to temperatures of at least 121 °C.

15. Process for providing a predetermined amount of a perfluorinated gas for a medical application from a gas storage device according to one of claims 1 to 6, wherein the amount of gas adsorbed on the adsorbent is released from the adsorbent contained in the gas storage device by thermal desorption or displacement desorption.

## Revendications

1. Réservoir de gaz pour le stockage d'une quantité de gaz prédéterminée d'un gaz perfluoré, comprenant un conteneur étanche aux gaz, dans lequel un adsorbant est intégré, l'adsorbant étant une zéolithe, sur laquelle à température ambiante, la quantité de gaz prédéterminée de gaz perfluoré et d'eau est adsorbée.

2. Réservoir de gaz selon la revendication 1, **caractérisé en ce que** l'adsorbant est une zéolithe du groupe des zéolithes désaluminées, de préférence, une zéolithe Y présentant un rapport de SiO2/Al2O3 > 200, en particulier un rapport de SiO2/Al2O3 > 1000.

3. Réservoir de gaz selon l'une des revendications 1 ou 2, **caractérisé en ce que** l'adsorbant est une zéolithe du groupe des MORDENITES présentant un rapport de SiO2/Al2O3 de 15 à 20.

4. Réservoir de gaz selon l'une des revendications 1 à 3, **caractérisé en ce que** les adsorbants présentent des tailles de pores de 0,2 à 1 nm et des surfaces de Langmuir > 400 m²/g.

5. Réservoir de gaz selon l'une des revendications 1 à 4, **caractérisé en ce que** le gaz stocké est un gaz perfluoré du groupe des SF₆, CF₄, C₂F₆ ou C₃F₈ ou un mélange de ces gaz.

6. Réservoir de gaz selon l'une des revendications 1 à 5, **caractérisé en ce que** la température de désorption du gaz est inférieure à la température de désorption de H₂O.

7. Utilisation d'un réservoir de gaz selon l'une des revendications précédentes, pour la stérilisation d'un gaz perfluoré stocké et la libération du gaz stérilisé pour une application médicale, le gaz adsorbé étant stérilisé par chauffage du réservoir de gaz et ensuite, libéré par désorption.

8. Utilisation d'un réservoir de gaz selon la revendication 7, **caractérisé en ce que** la pression de désorption de l'adsorbant chargé de gaz à 121° C ne dépasse pas 3,5 bars dans un réservoir fermé, lorsque le réservoir est rempli d'adsorbant à la moitié de son volume.

9. Utilisation selon l'une des revendications 7 ou 8, **caractérisée en ce que** la désorption du gaz s'effectue par chauffage de l'adsorbant à un niveau supérieur à la température de désorption du gaz.

10. Utilisation selon l'une des revendications 7 à 9, **caractérisée en ce que** le gaz pour l'application médicale prévue est libéré par chauffage et est intégré dans un agent d'application.

11. Utilisation selon l'une des revendications 7 à 10, **caractérisée en ce que** la libération du gaz adsorbant s'effectue par adsorption d'une substance avec une affinité d'adsorption supérieure à celle du gaz adsorbé sur l'adsorbant, ce qui entraîne une désorption de gaz par turbulence.

12. Utilisation selon l'une des revendications 7 à 11, **caractérisée en ce que** le gaz est désorbé par désorption thermique de l'adsorbeur en introduisant un additif dans le réservoir de gaz qui adsorbe l'adsorbeur, une quantité de chaleur d'adsorption étant libérée, entraînant une désorption du gaz.

13. Utilisation selon l'une des revendications 7 à 12, **caractérisée en ce que** pour le prélèvement de gaz, le gaz est désorbé par désorption par turbulence de l'adsorbeur, en introduisant un additif dans le réservoir de gaz qui présente pour l'adsorbant utilisé, une affinité d'adsorption supérieure à celle du gaz prévu pour l'application médicale.

14. Utilisation selon l'une des revendications 7 à 13, **caractérisée par** les étapes suivantes :
- chauffage de l'adsorbant pour éliminer les impuretés et pour activer celui-ci,
- adsorption du gaz sur l'adsorbant et addition d'eau,
- renfermement de l'adsorbant adsorbé avec le gaz dans un réservoir étanche aux gaz,
- stérilisation du gaz et de l'adsorbant dans le réservoir par chauffage du réservoir à des températures d'au moins 121°C.

15. Procédé de préparation d'une quantité prédéterminée d'un gaz perfluoré pour une application médicale à partir d'un réservoir de gaz selon l'une des revendications 1 à 6, dans lequel la quantité de gaz adsorbée sur l'adsorbant est libérée par désorption thermique ou désorption par turbulence de l'adsorbant contenu dans le réservoir de gaz.
